# EUROPEAN PATENT SPECIFICATION

(11) **EP 2 366 333 B1**
(45) Date of publication and mention of the grant of the patent: **05.03.2014**
(21) Application number: 11158290.4
(22) Date of filing: 15.03.2011
(51) Int. Cl.: A61B 6/00, A61B 8/08

(54) **Medical imaging device comprising radiographic acquisition means and guide means for ultrasound probe**
Medizinische Bildgebungsvorrichtung mit einem Röntgengerät und Mitteln zur Führung einer Ultraschallsonde
Dispositif d'imagerie médicale comprenant des moyens d'acquisition radiographique et des moyens de guidage d'une sonde ultrasonore

(30) Priority: 17.03.2010 FR 1051897
(43) Date of publication of application: 21.09.2011
(73) Proprietor: General Electric Company, Schenectady, NY 12345 (US)
(72) Inventor: Muller, Serge Louis Wilfrid, 78533, Buc (FR)
(74) Representative: Bedford, Grant Richard

(56) References cited:
- DE-A1-102007 007 386
- FR-A1- 2 862 519
- FR-A1- 2 875 694
- US-A1- 2003 149 364
- US-A1- 2007 167 806
- US-A1- 2009 024 030

## Description

The present invention relates generally to the field of medical imaging. In particular, the present invention relates to a medical imaging device applicable especially though not exclusively to mammography.

Radiography and echography are two imaging techniques used for exploration of the breast.

Radiography produces an image of internal structures of an object from X-rays passing through these internal structures.

Echography produces an image of the internal structures of an object from ultrasound reflected by said internal structures.

It is known today that radiography and echography are two complementary techniques used for examining the breast.

Medical imaging devices combining these two imaging techniques have therefore already been proposed.

For example, the document US 5,938,613 describes an apparatus combining X-ray mammography equipment with an ultrasound transducer. This apparatus generates images of the internal structure of breast tissue from X-ray and ultrasound. The transducer is mounted on a sled which moves by means of a motor and is carried out in increments. The sled moves on a transparent X-ray and ultrasound compression plate of the breast. This apparatus has drawbacks. Shifting the sled in increments limits possibilities for generating images from the transducer. The sled on which the transducer is mounted moves on the compression plate and takes images only according to the direction perpendicular to the compression plate. The user cannot move the transducer as rapidly and freely as preferred and cannot conduct conventional examination via ultrasound. If the user wants to take images via ultrasound according to a direction transverse to this direction, the patient must change position.

Imaging apparatus comprising an X-ray mammography system and an ultrasound probe capable of being moved manually by a user has also been proposed in the document FR 2,835,731. This apparatus comprises a positioning probe of the ultrasound probe. This imaging apparatus more easily matches mammographic images and echographic images. However, it is difficult for the user to obtain mammographic and echographic images of the same zone of the breast using the apparatus described in FR 2,835,731.

US 2009/024030 discusses methods and systems for guiding the acquisition of ultrasound images.

FR 2 862 519 relates to a compliant probe interface assembly for an automated medical imaging system.

An aim of the invention is to propose a device for taking diverse images of a given zone of the breast by X-ray and by ultrasound using the same acquisition geometry for acquisition via X-ray and ultrasound.

For this purpose, an imaging device according to the present invention as defined in appended claim 1 is provided.

In this way, the invention helps the user in shifting the ultrasound probe to a zone on the object to be imaged via echography.

Preferred though non-limiting aspects of embodiments of the invention are the following: the guide means comprise a positioning probe of the ultrasound probe, said guide means being adapted to send information on the position of the probe to the processing means, the processing means are adapted to display the path between the position of the ultrasound probe and the position in the object corresponding to the selected zone for each new position of the probe, the processing means are adapted to determine a volume in which the path is registered, the guide means comprise a haptic arm with power return.

An aspect of the invention also relates to a medical imaging process as defined in appended claim 5.

Preferred though non-limiting aspects of the process according to this aspect of the invention are the following: the process further comprises receiving information on the position of the ultrasound probe, the process further comprises, for each new position of the probe, disclosing the path between the position of the probe and the position in the object corresponding to the selected zone.

An aspect of the invention also relates to a programmed computer product comprising programmed code instructions for executing the process described hereinabove.

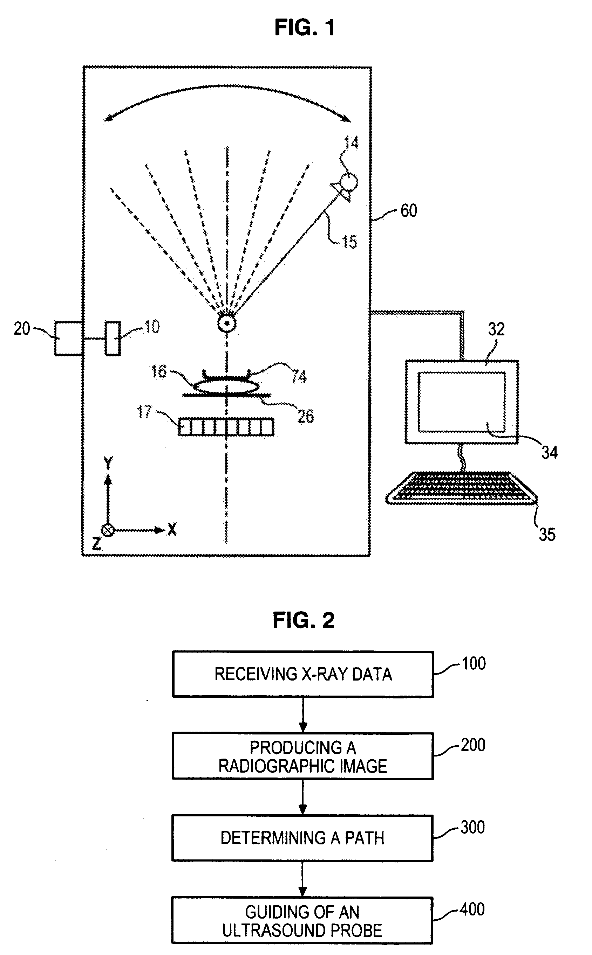

Various characteristics and advantages of a device and of a process according to various aspects of the invention will emerge from the following description which is purely illustrative and non-limiting and which is considered in conjunction with the attached diagrams, in which Figures 1 to 3 are schematic representations of a device and an imaging process according to various embodiments of the invention.

Aspects and embodiments of the invention will now be described in greater detail in reference to Figures 1 to 3. In the following description, the object 16 to be imaged is a breast of a patient. However, it is understood that the present invention can be applied to imaging of other objects.

Figure 1 represents a medical imaging device comprising X-ray acquisition means, an ultrasound probe 10, processing means 32 and guide means 20.

The X-ray acquisition means comprise an arm 15 containing a radiation source 14 for emitting X-rays, a radiation detector 17 for receiving the X-rays, a planar object support 26 placed between the source 14 and the detector 17, a pad 74 placed between the object support 26 and the source 14 for compression of the object 16 to be imaged. The compression pad 74 is transparent to X-ray and ultrasound. The arm 15 bearing the source 14 can be moved into a plurality of positions 15. This arm 15 plays the role of positioner. As for the source 14, it is mounted pivoting on the arm 15 to allow orientation of the latter relative to the object support 26.

The ultrasound probe 10 enables emission and receiving of ultrasound. The ultrasound probe 10 is capable of being shifted manually by a user. A 2D probe 10 for acquiring a set of 2D data on the breast 16 in the form of cups can be used. Interpolation of this 2D data set generates 3D reconstruction of the structure of the breast tissue. A 3D (or even 4D) probe 10 for acquiring a data set having 3D reconstruction of the structure of the breast tissue can also be used.

The processing means 32 are for receiving data sent by the X-ray acquisition means on the one hand. The processing means are able to produce a plurality of radiographic projections each corresponding to a respective position of the radiation source from the data received from the acquisition means. The processing means 32 are also able to utilise reconstruction processes for obtaining 3D information from radiographic projections. The processing means 32 are also able to receive the data emitted by the ultrasound probe. The processing means 32 are adapted to produce one (or more) echographic image(s) from these data.

The guide means 20 guide the shifting of the ultrasound probe 10, as will be explained in greater detail hereinbelow. The guide means 20 are a haptic arm at the end of which the ultrasound probe is fixed. The haptic arm preferably has a power return. It should be remembered the term *"haptic* "designates any technology for touching and manipulating virtual and/or distant objects. Use of a haptic arm "slides" the ultrasound probe 10 on the surface of a virtual volume produced by the processing means. The haptic arm can be integrated in or separate from the X-ray acquisition means. In all cases, the position of the haptic arm is known. A calibrating procedure determines the mathematical transformation for moving from the reference of the acquisition means to the reference of the haptic arm.

Various aspects and embodiments of the invention are based on the following statement.

When a user detects a region of interest in a mammograph, he may want to obtain extra information on this region by using an ultrasound probe to make an echographic image of the region of interest. Actually, the echographic image is likely to provide the user with extra information helping him confirm diagnosis.

Yet, with the devices of the prior art, locating a region in the breast is difficult. In fact, the user has only the mammographic image for finding the suspect region to then produce the echographic image. This causes a significant increase in the length of examination.

Due to the time necessary for finding the suspect region to then produce the echographic image, it is frequently necessary to remove the pad, with compression exerted by the latter being a source of discomfort for the patient. This causes a variation in the geometric configuration of the breast which can cause errors in matching the echographic and mammographic images.

Certain aspects of the invention propose a solution for orienting the user in positioning the ultrasound probe 10 on a breast 16 which remains compressed throughout examination. In this way, the geometric configuration of the breast is identical during acquisition of the radiological and echographic images, which lowers the risk of error during matching of these images.

In fact, the presence of guide means 20 in the imaging device allows the user to more quickly find a suspect region identified in the mammographic image, and therefore more quickly and easily take images using the ultrasound probe. It is no longer necessary to remove the pad, allowing mammographic and echographic images to be arranged in which geometric configuration of the breast is identical.

The operating principle is the following. Once the object to be imaged is positioned on the object support and compressed by the pad, data representative of the object are acquired using the X-ray acquisition means.

The processing means receive 100 these data, and produce 200 radiographic images of the object (in 2D or 3D) from the acquired data.

The radiographic images are used for selecting one (or more) region(s) of interest in the breast. This locating can be done manually by the user using gripping means of the device. This locating can also be done automatically by the processing means using a radiological image-analysis process known to the person skilled in the art - for example CAD software (acronym for "Computer Aided Detection"). The region of interest can be a point, trimming or a predefined form.

Knowing the position of the region of interest in the radiographic images, the processing means determine 300 a path 30 between the position of the ultrasound probe and a position in the breast corresponding to the selected region of interest. More precisely, from the position of the region of interest in the radiographic images, the processing means 32 determine the position of this region in the breast (the geometric configuration of the breast and the geometry of the acquisition means being known). Also, since the geometry of the haptic arm is known, the position of the probe which is fixed at its end is also known. The processing means therefore have sufficient information for determining a path between the position of the ultrasound source and the position in the breast corresponding to the selected region of interest.

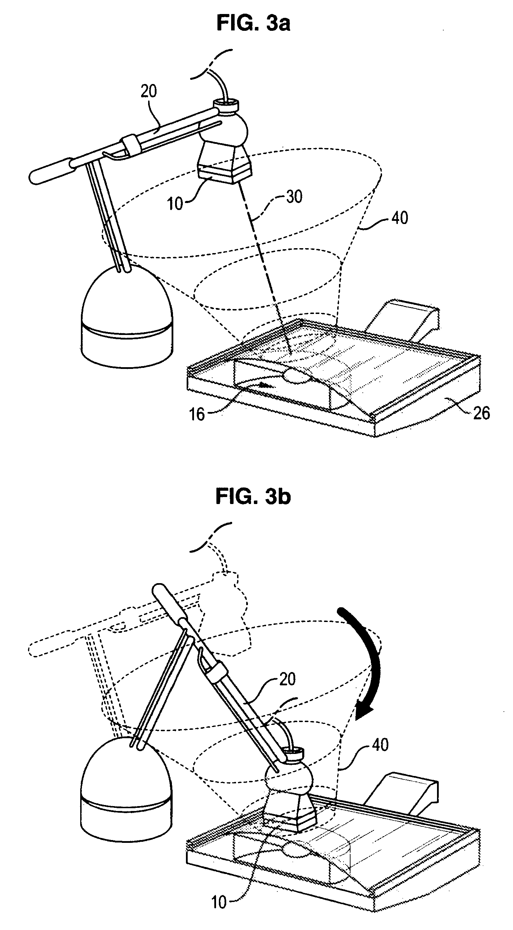

The processing means determines a virtual volume 40 in which the path is registered. This virtual volume 40 guides the movement of the user to move the probe to the breast of the patient, as illustrated in Figures 3a and 3b. This virtual volume can have different forms. The form of the virtual volume can be for example a truncated cone, or a truncated torus. The virtual volume has a funnel shape so as to progressively channel shifting of the probe to the position of the region of interest in the breast, but also to orient the ultrasound probe according to an axis parallel to the X-ray beam emitted by the acquisition means.

In this way, various embodiments of invention utilise radiographic images to construct a virtual volume which limits shifting of the ultrasound probe connected to a haptic device such that the user is guided 400 as far as the position on the breast where the ultrasound probe will be capable of displaying the selected region of interest on radiographic images. This reduces the duration of the examination.

In a variant embodiment, the processing means 32 are able to display the path 30 for each new position of the ultrasound source. This defines a new path and a new virtual volume, especially assuming that the user has shifted the ultrasound probe out of the virtual volume.

The reader will have understood that numerous modifications can be made without departing materially from the new ideas and advantages described here.

For example, in the case where the region of interest is a point, a target region can be determined by the processing means. This target region is obtained for example by using region-enhancing processes or any other process known to the person skilled in the art.

Similarly, the guide means can be types other than a haptic arm. For example, the guide means can be lighting means adapted to illuminate the position on the breast of the patient corresponding to the selected region of interest in the radiographic images.

Consequently, all modifications of this type are intended to be incorporated within the scope of the system and imaging process such as defined in the attached claims.

## Claims

1. A medical imaging device, comprising:
X-ray acquisition means (14, 15, 17, 26, 74) adapted for acquisition of data representative of an object (16),
an ultrasound probe (10) capable of being shifted manually by a user,
a compression pad (74) for compressing the object (16) to be imaged, said compression pad (74) being transparent to X-rays and ultrasound;
processing means (32) adapted to:
produce at least one image of the object from data acquired by the X-ray acquisition means, and
determine a path (30) between a position of the ultrasound probe (10) and a position in the object (16) corresponding to a selected zone in said image; and
guide means (20) for the ultrasound probe (10), said guide means comprising a haptic arm being adapted to facilitate shifting the probe along the path between a position of the ultrasound probe (10) and the position in the object (16) corresponding to the selected zone in said image; and **characterised in that**
the processing means (32) are adapted to determine a virtual volume (40) in which the path is registered said virtual volume (40) having a funnel shape to enable progressive shifting of the ultrasound probe (10) to the position of a region of interest in said object (16) and to orient the ultrasound probe (10) to an axis parallel to an X-ray beam emitted by the X-ray acquisition means (14, 15, 17, 26, 74).

2. The device as claimed in the preceding claim, in which the guide means (20) comprise a positioning probe of the ultrasound probe (10), said guide means (20) being adapted for sending information on the position of the probe to the processing means.

3. The device as claimed in any preceding claim, wherein the processing means (32) are adapted to display the path between the position of the ultrasound probe (10) and the position in the object (16) corresponding to the selected zone for each new position of the probe.

4. The device as claimed in any preceding claim, in which the guide means (20) comprise a haptic arm with power return.

5. A medical imaging process, comprising the steps of:
compressing an object (16) to be imaged using a compression pad (74) that is transparent to X-rays and ultrasound;
receiving (100) data representative of the object (16) acquired by X-ray acquisition means (14),
processing (200) data, by processing means (32), to produce at least one image of the object (16),
determining (300), via the processing means (32), a path between an ultrasound probe (10) position and a position in the object (16) corresponding to a selected zone in the image,
guiding (400), by guide means (20) comprising a haptic arm, the ultrasound probe (10) to facilitate shifting the probe along the path between a position of the ultrasound probe (10) and a position in the object (16) corresponding to the selected zone in said image **characterised in that** the processing means (32) are adapted to determine a virtual volume (40) in which the path is registered said virtual volume (40) having a funnel shape to enable progressive shifting of the ultrasound probe (10) to the position of a region of interest in said object (16) and to orient the ultrasound probe (10) to an axis parallel to an X-ray beam emitted by the X-ray acquisition means (14, 15, 17, 26, 74).

6. The process as claimed in claim 5, which further comprises receiving information on the position of the ultrasound probe (10).

7. The process as claimed in claim 5 or claim 6, which further comprises, for each new position of the probe (10), displaying the path between the position of the probe (10) and the position in the object (16) corresponding to the selected zone.

8. A computer program product, comprising code instructions programmed to implement the process as claimed in any one of Claims 5 to 7.

9. The process as claimed in any one of claims 6 to 8, which further comprises determining a volume (40) in which the path is registered.

10. A computer program product, comprising code instructions programmed to implement the process as claimed in any one of Claims 6 to 9.

## Patentansprüche

1. Eine medizinische Bildgebungsvorrichtung, umfassend:
Röntgenerfassungsmittel (14, 15, 17, 26, 74), die geeignet zum Erfassen von Daten sind, die für ein Objekt (16) kennzeichnend sind,
eine Ultraschallsonde (10), die dazu fähig ist durch einen Verwender manuell verschoben zu werden,
eine Komprimierplatte (74) zum Verdichten des Objekts (16), das abgebildet werden soll, wobei die Komprimierplatte (74) durchlässig für Röntgen und Ultraschall ist;
Verarbeitungsmittel (32), die geeignet sind, um:
mindestens ein Bild eines Objekts von den Daten, die durch die Röntgenerfassungsmittel erfasst wurden, herzustellen, und
Bestimmen einer Bahn (30) zwischen einer Position der Ultraschallsonde (10) und einer Position in dem Objekt (16), die zu einem ausgewählten Bereich in dem Bild korrespondiert; und
Führungsmittel (20) für die Ultraschallprobe (10), wobei das Führungsmittel einen haptischen Arm umfasst, der geeignet ist, um das Verschieben der Sonde entlang der Bahn zwischen einer Position der Ultraschallsonde (10) und der Position in dem Objekt (16), die zu dem ausgewählten Bereich in dem Bild korrespondiert, zu unterstützen; und **dadurch gekennzeichnet ist, dass**
die Verarbeitungsmittel (32) geeignet sind, um ein virtuelles Volumen (40) zu bestimmen, in welchem die Bahn angezeigt wird, wobei das virtuelle Volumen (40) eine Trichterform aufweist, um ein fortschreitendes Verschieben der Ultraschallsonde (10) zu der Position des Bereichs von Interesse in dem Objekt (16) zu ermöglichen und die Ultraschallsonde (10) nach einer Achse auszurichten, die parallel zu einem Röntgenstrahl, der durch die Röntgenerfassungsmittel (14, 15, 17, 26, 74) emittiert wird.

2. Die Vorrichtung wie in dem vorherigen Anspruch beansprucht, in welchem das Führungsmittel (20) eine Positionierungssonde der Ultraschallsonde (10) umfasst, wobei das Führungsmittel (20) geeignet ist, um Informationen über die Position der Sonde zu dem Verarbeitungsmittel zu senden.

3. Die Vorrichtung wie in einem vorherigen Anspruch beansprucht, wobei die Verarbeitungsmittel (32) geeignet sind, die Bahn zwischen der Position der Ultraschallsonde (10) und der Position in dem Objekt (16), die zu dem ausgewählten Bereich für jede neue Position der Sonde korrespondiert, anzuzeigen.

4. Die Vorrichtung wie in einem vorherigen Anspruch beansprucht, in welcher die Führungsmittel (20) einen haptischen Arm mit Spannungswiederkehr umfassen.

5. Ein medizinisches Bildgebungsverfahren, umfassend die Schritte:
Komprimieren eines Objekts (16), das abgebildet werden soll, unter Verwenden einer Komprimierplatte (74), die für Röntgen und Ultraschall durchlässig ist;
Empfangen (100) von Daten, die für das Objekt (16) charakteristisch sind, die durch Röntgenerfassungsmittel (14) erlangt wurden,
Verarbeiten (200) der Daten, durch die Verarbeitungsmittel (32), um mindestens ein Bild des Objekts (16) herzustellen,
Bestimmen (300), mittels des Verarbeitungsmittels (32) einer Bahn zwischen einer Ultraschallsondenposition (10) und einer Position in dem Objekt (16), die zu einem ausgewählten Bereich in dem Bild korrespondiert,
Führen (400), durch Führungsmittel (20), die einen haptischen Arm umfassen, der Ultraschallsonde (10), um Verschieben der Sonde entlang der Bahn zwischen einer Position der Ultraschallsonde (10) und einer Position in dem Objekt (16), die zu dem ausgewählten Bereich korrespondiert, zu unterstützen, **dadurch gekennzeichnet, dass** die Verarbeitungsmittel (32) geeignet sind, um ein virtuelles Volumen (40) zu bestimmen, in welchem die Bahn angezeigt wird, wobei das virtuelle Volumen (40) eine Trichterform aufweist, um fortschreitendes Verschieben der Ultraschallsonde (10) zu der Position einer Region von Interesse in dem Objekt (16) zu ermöglichen und um die Ultraschallsonde (10) nach einer Achse auszurichten, die parallel zu einem Röntgenstrahl ist, der durch ein Röntgenerfassungsmittel (14, 15, 17, 26, 74) emittiert wird.

6. Ein Verfahren wie in Anspruch 5 beansprucht, welches ferner Empfangen von Informationen über die Position der Ultraschallsonde (10) umfasst.

7. Das Verfahren wie in Anspruch 5 oder Anspruch 6 beansprucht, welches ferner für jede neue Position der Sonde (10), Darstellen der Bahn zwischen der Position der Sonde (10) und der Position in dem Objekt (16), die zu dem ausgewählten Bereich korrespondiert, umfasst.

8. Ein Computerprogrammerzeugnis, umfassend Codebefehle, die programmiert sind, um das Verfahren, wie in einem der Ansprüche 5 bis 7 beansprucht, zu implementieren.

9. Das Verfahren wie in einem der Ansprüche 6 bis 8 beansprucht, welches ferner das Bestimmen eines Volumens (40), in welchem die Bahn angezeigt ist, umfasst.

10. Ein Computerprogrammerzeugnis, umfassend Codebefehle, die programmiert sind, um das Verfahren, wie in einem der Ansprüche 6 bis 9 beansprucht, zu implementieren.

## Revendications

1. Dispositif d'imagerie médicale, comprenant :
des moyens à'acquisition radiographiques (14, 15, 17, 26, 74) adaptés à une acquisition de données représentatives d'un objet (16),
une sonde ultrasonore (10) qui est à même d'être déplacée manuellement par un utilisateur,
une plaquette de compression (74) pour comprimer l'objet (16) qui doit être imagé, ladite plaquette de compression (74) étant transparente aux rayons X et aux ultrasons ;
des moyens de traitement (32) qui sont à même de :
produire au moins une image de l'objet à partir de données acquises par les moyens d'acquisition radiographiques, et
déterminer un trajet (30) entre une position de la sonde ultrasonore (10) et une position dans l'objet (16) correspondant à une zone sélectionnée dans ladite image ; et
des moyens de guidage (20) pour la sonde ultrasonore (10), lesdits moyens de guidage comprenant un bras haptique qui est à même de faciliter le déplacement de la sonde le long du trajet entre une position de la sonde ultrasonore (10) et la position dans l'objet (16) correspondant à la zone sélectionnée dans ladite image ; et
**caractérisé en ce que** :
les moyens de traitement (32) sont à même de déterminer un volume virtuel (40) dans lequel le trajet est enregistré, ledit volume virtuel (40) ayant une forme d'entonnoir pour permettre un déplacement progressif de la sonde ultrasonore (10) dans la position d'une région intéressante dudit objet (16) et pour orienter la sonde ultrasonore (10) selon un axe parallèle à un faisceau de rayons X émis par les moyens d'acquisition radiographiques (14, 15, 17, 26, 74).

2. Dispositif selon la revendication précédente, dans lequel les moyens de guidage (20) comprennent une sonde de positionnement de la sonde ultrasonore (10), lesdits moyens de guidage (20) étant à même d'envoyer des informations sur la position de la sonde aux moyens de traitement.

3. Dispositif selon l'une quelconque des revendications précédentes, dans lequel les moyens de traitement (32) sont à même d'afficher le trajet entre la position de la sonde ultrasonore (10) et la position dans l'objet (16) correspondant à la zone sélectionnée pour chaque nouvelle position de la sonde.

4. Dispositif selon l'une quelconque des revendications précédentes, dans lequel les moyens de guidage (20) comprennent un bras haptique avec un retour de puissance.

5. Procédé d'imagerie médicale, comprenant les étapes consistant à :
comprimer un objet (16) à imager en utilisant une plaquette de compression (74) qui est transparente aux rayons X et aux ultrasons ;
recevoir (100) des données représentatives de l'objet (16) acquises par les moyens d'acquisition radiographiques (14),
traiter (200) des données par des moyens de traitement (32) pour produire au moins une image de l'objet (16),
déterminer (300), via les moyens de traitement (32), un trajet entre la position d"une sonde ultrasonore (10) et une position dans l'objet (16) correspondant à une zone sélectionnée de l'image,
guider (400), par des moyens de guidage (20) comprenant un bras haptique, la sonde ultrasonore (10) pour faciliter le déplacement de la sonde le long du trajet entre une position de la sonde ultrasonore (10) et une position dans l'objet (16) correspondant à la zone sélectionnée dans ladite image, **caractérisé en ce que** les moyens de traitement (32) sont à même de déterminer un volume virtuel (40), dans lequel le trajet est enregistré, ledit volume virtuel (40) ayant une forme en entonnoir pour permettre un déplacement progressif de la sonde ultrasonore (10) dans la position d'une région intéressante dans ledit objet (16) et pour orienter la sonde ultrasonore (10) selon un axe parallèle à un faisceau de rayons X émis par les moyens d'acquisition radiographiques (14, 15, 17, 26, 74).

6. Procédé selon la revendication 5, qui comprend en outre la réception d'informations sur la position de la sonde ultrasonore (10).

7. Procédé selon la revendication 5 ou la revendication 6, qui comprend en outre, pour chaque nouvelle position de la sonde (10), l'affichage du trajet entre la position de la sonde (10) et la position dans l'objet (16) correspondant à la zone sélectionnée.

8. Produit de programme informatique comprenant des instructions de codage programmées pour mettre en oeuvre le procédé selon l'une quelconque des revendications 5 à 7.

9. Procédé selon l'une quelconque des revendications 6 à 8, qui comprend en outre la détermination d'un volume (40) dans lequel le trajet est enregistré.

10. Produit de programme informatique comprenant des instructions de codage programmées pour mettre en oeuvre le procédé selon l'une quelconque des revendications 6 à 9.
